## Europäisches Patentamt
## European Patent Office
## Office européen des brevets

(19)

(11) Veröffentlichungsnummer: **0 245 527 B1**

# EUROPÄISCHE PATENTSCHRIFT

(12)

(45) Veröffentlichungstag der Patentschrift: **27.03.91**

(51) Int. Cl.⁵: **A61F 2/34**

(21) Anmeldenummer: **86106098.6**

(22) Anmeldetag: **03.05.86**

---

(54) Endoprothese für eine Hüftgelenkspfanne.

---

(30) Priorität: **15.04.86 CH 1490/86**

(43) Veröffentlichungstag der Anmeldung:
**19.11.87 Patentblatt 87/47**

(45) Bekanntmachung des Hinweises auf die
Patenterteilung:
**27.03.91 Patentblatt 91/13**

(84) Benannte Vertragsstaaten:
**AT DE FR GB IT**

(56) Entgegenhaltungen:
**EP-A- 0 139 356**
**FR-A- 2 210 909**
**GB-A- 2 001 247**
**GB-A- 2 013 503**
**GB-A- 2 159 416**

(73) Patentinhaber: **GEBRÜDER SULZER AKTIENGE-
SELLSCHAFT**
**Zürcherstrasse 9**
**CH-8401 Winterthur(CH)**

(72) Erfinder: **Zweymüller, Karl, Prof. Dr.-med.**
**Döblinger Hauptstrasse 22/5**
**A-1190 Wien(AT)**

---

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die Erfindung betrifft eine Endoprothese für eine Hüftgelenkspfanne, bestehend aus einer im Knochen verankerbaren Aussenschale und einem inneren Pfannenkörper aus Kunststoff, wobei der Pfannenkörper mit einem konischen Mantel und die Aussenschale mit einem an die Mantelform des Pfannenkörpers angepassten Hohlraum versehen und Pfannenkörper und Aussenschale durch einen Schnappverschluss, bei dem ein Vorsprung im Mantel des Pfannenkörpers in eine Vertiefung der Hohlraumwand eingreift, ineinander gehalten sind.

Eine Prothese der vorstehend genannten Art ist beispielsweise bekannt aus der EP-A-0 065 482, der DE-C-24 11 617 oder der FR-A-2 519 545; für die Verankerung im Beckenknochen wird die Aussenschale der Prothese nach der erstgenannten Druckschrift zunächst mit Hilfe eines Werkzeugs in Richtung ihrer Achse in eine operativ geschaffene Ausnehmung im Beckenknochen eingesetzt. Dann wird der Pfannenkörper eingetrieben, der durch einen Schnappverschluss in der Aussenschale fixiert wird.

Eine ähnliche Konstruktion zeigt die GB-A 2 159 416; der Mantel des Kunststoff-Pfannenkörpers ist bei einer der dortigen Konstruktionen mit zwei im axialen Abstand angeordneten, wulstartig hervorstehenden Ringen versehen, die über den ganzen Umfang durchgehend verlaufen und in entsprechende Ringnuten im Hohlraum der Aussenschale einrasten.

Bei allen diesen bekannten Konstruktionen können in der Praxis beim Eintreiben des Pfannenkörpers Probleme auftreten, da es schwierig ist, den Pfannenkörper in die bereits "gesetzte" Aussenschale einzutreiben, ohne dass es zu einem Verkanten des Pfannenkörpers relativ zur Achse der Schale kommt, was Beschädigungen des Pfannenkörpers zur Folge haben kann.

Aufgabe der Erfindung ist es somit, eine Prothese der eingangs genannten Art zu schaffen, bei der das Einsetzen des Pfannenkörpers in die Aussenschale durchgeführt werden kann, ohne dass ein Verkanten der beiden Elemente relativ zueinander möglich ist.

Diese Aufgabe wird mit der Erfindung dadurch gelöst, dass im Abstand vom nahe dem Aequator angeordneten Schnappverschluss gegen den Pol hin versetzt im Mantel des Pfannenkörpers ein in Umfangsrichtung verlaufender, über den ganzen Umfang durchgehender Nocken angeordnet ist, und dass im Hohlraum der Aussenschale eine zugehörige und als Führung für den Nocken wirkende Nut angeordnet ist, die eine parallel zur Symmetrieachse der Pfanne verlaufende Zylindermantelfläche aufweist, wobei bei einem Einschieben des Pfannenkörpers in die Aussenschale in Richtung der Pfannenachse der Nocken in die Nut eingreift, bevor der Vorsprung an der Hohlraumwand der Aussenschale zum Aufweiten der Aussenschale zur Anlage kommt.

In ihrem Zusammenwirken bilden die Nut in der Aussenschale und der Nocken eine längs des ganzen Umfangs wirksame Führung für den Pfannenkörper im Hohlraum der Aussenschale, insbesondere ab dem Punkt des Einsetzweges, an dem der Vorsprung des Schnappverschlusses beginnt, die Aussenschale aufzuweiten, wobei die parallel zur Symmetrieachse der Pfanne verlaufende Zylindermantelfläche die Führung in Richtung der Pfannenachse gewährleistet.

Bei dem erwähnten Aufweiten der Aussenschale durch den Pfannenkörper besteht die Gefahr, dass der Pfannenkörper-Vorsprung, der in die Vertiefung der Hohlraumwand der Aussenschale eingreifen soll, durch die notwendigen "Aufweitkräfte" teilweise plastisch verformt wird, so dass der Kunststoff zu fliessen beginnt. Zusätzlich ist es daher zweckmässig, wenn die äquatorseitige Begrenzung der Vertiefung in der Aussenschale senkrecht zur Symmetrieachse der Prothese verläuft, während der zugehörige Abschluss des Vorsprungs mit dieser Begrenzung einen spitzen Winkel bildet. Zwischen Vertiefung und Vorsprung entsteht auf diese Weise ein Hohlraum zur Aufnahme des verformten Kunststoffes.

Im folgenden wird die Erfindung anhand eines Ausführungsbeispiels im Zusammenhang mit der Zeichnung näher erläutert.

Fig. 1 und 2     zeigen in je einem Ausschnitt aus Radial- oder Meridianschnitten durch die neue Prothese den äquatornahen Randbereich von Aussenschale und Pfannenkörper, wobei Fig. 1 die beiden Elemente während des Einsetzens des Pfannenkörpers wiedergibt, während Fig. 2 den Endzustand zeigt;

Fig. 3     ist ein Detail D aus Fig. 2 in vergrössertem Massstab.

Die vorzugsweise aus Metall, z.B. aus Titan oder einer Titanlegierung, bestehende Aussenschale 1, die zementfrei im Beckenknochen verankert, z.B. eingepresst oder eingeschraubt, werden kann, nimmt in ihrem Hohlraum 2 einen Pfannenkörper 3 auf. Dieser beteht aus Kunststoff, beispielsweise aus Polyäthylen, und enthält die eigentliche Gelenkpfanne 4, in die ein nicht gezeigter Gelenkkopf einer Femurkopfprothese eingreifen soll.

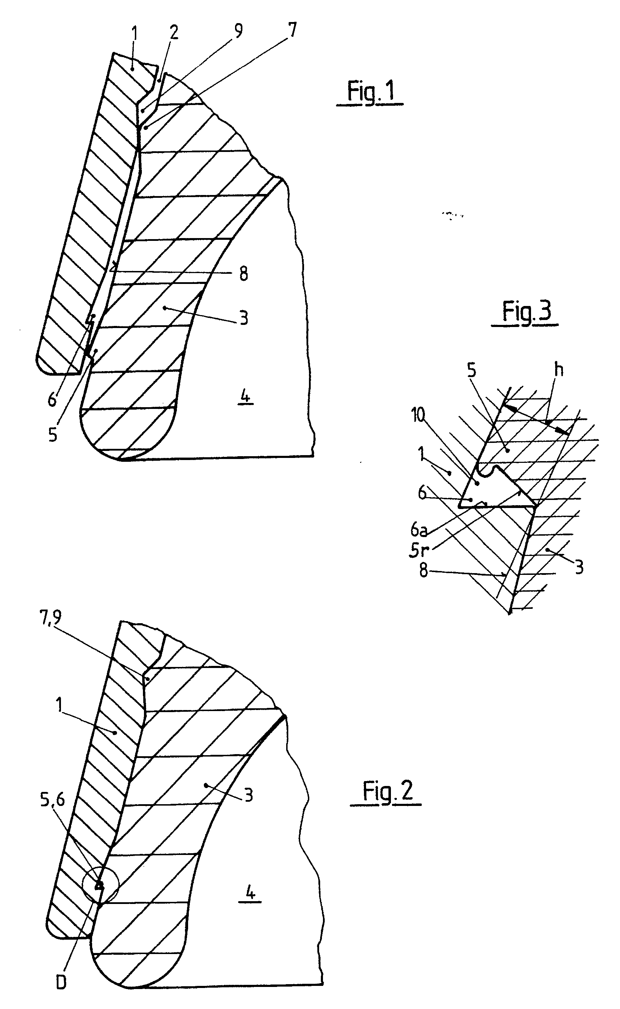

Die Verbindung der beiden Teile 1 und 3 erfolgt nahe dem äquatorialen Rand der Prothese mit Hilfe eines Schnappverschlusses 5, 6, bei dem ein aus dem Mantel des Pfannenkörpers 3 in Richtung vom Pol zum Aequator "herauswachsender" Vorsprung 5 in eine als entsprechendes Gegenstück geformte Vertiefung 6 in der Hohlraumwand der Aussenschale 1 einschnappt (Fig. 2).

Erfindungsgemäss ist polwärts des Schnappverschlusses 5, 6 eine Führung zwischen Pfannenkörper 3 und Aussenschale 1 vorgesehen. Diese Führung soll bewirken, dass der Pfannenkörper 2 beim Eintreiben oder Einpressen in die bereits im Knochen befestigte Aussenschale 1 geführt wird. Die Führung besteht aus einem Nocken 7, der in Umfangsrichtung durchgehend aus der Mantelfläche 8 des Pfannenkörpers 3 hervorsteht, und aus einer entsprechenden Nut 9, die in die Wand des Aussenschalenhohlraumes 2 eingeschnitten ist. In dem gezeigten Beispiel sind Nocken 7 und Nut 9 Zylindermantelflächen von parallel und konzentrisch zur nicht gezeigten Symmetrieachse der Prothese verlaufenden Kreiszylindern. Wie erwähnt, vereinfacht dies Zylinderform der Nut 9 den Herstellungsaufwand erheblich.

Wie ebenfalls bereits erwähnt, ist die Führung des Pfannenkörpers 3 in der Aussenschale 1 beim Eintreibprozess für den Pfannenkörper 1 von dem Punkt ab besonders wichtig, an dem der Vorsprung 5 beginnt, die Aussenschale 1 aufzuweiten. Die Führung muss also spätestens in diesem Punkt einsetzen, wozu der Abstand der Nut 9 vom äquatorialen Rand der Aussenschale 1 und der Abstand des Nockens 7 vom Vorsprung 5 des Schnappverschlusses auf der Mantelfläche 8 des Pfannenkörpers 3 - unter Berücksichtigung der Höhe h (Fig. 3) des Vorsprungs 5 und des Neigungswinkels von Aussenschale 1 und Mantelfläche 8 zur vertikalen Symmetrieachse der Prothese - entsprechend gewählt werden.

Der "Spitzen"-Bereich des Vorsprungs 5 erleidet beim Einpressen des Pfannenkörpers 3 in die relativ harte metallene Aussenschale 1 eine bleibende Verformung. Um einen Raum für die Aufnahme des Materials dieser Verformung zu schaffen, ist es zweckmässig, wenn die dem Aequator zugewandte Begrenzung 6a (Fig. 3) der Vertiefung 6 senkrecht zur nicht gezeigten Symmetrieachse der Prothese verläuft, während der zugehörige Rand 5r des Vorsprungs 5 unter einem spitzen Winkel - im gezeigten Beispiel von 45° - dazu verläuft. Wie Fig. 3 erkennen lässt, entsteht so ein dreieckiger Hohlraum 10, in den das verdrängte Kunststoffmaterial des Vorsprungs 5 "hineinfliessen" kann.

## Ansprüche

1.  Endoprothese für eine Hüftgelenkspfanne, bestehend aus einer im Knochen verankerbaren Aussenschale (1) und einem inneren Pfannenkörper (3) aus Kunststoff, wobei der Pfannenkörper (3) mit einem konischen Mantel und die Aussenschale (1) mit einem an die Mantelform des Pfannenkörpers (3) angepassten Hohlraum (2) versehen, und Pfannenkörper (3) und Aussenschale (1) durch einen Schnappverschluss (5, 6), bei dem ein Vorsprung (5) im Mantel des Pfannenkörpers (3) in eine Vertiefung (6) der Hohlraumwand eingreift, ineinander gehalten sind, dadurch gekennzeichnet, dass im Abstand vom nahe dem Aequator angeordneten Schnappverschluss (5, 6) gegen den Pol hin versetzt im Mantel (8) des Pfannenkörpers (3) ein in Umfangsrichtung verlaufender, über den ganzen Umfang durchgehender Nocken (7) angeordnet ist, und dass im Hohlraum (2) der Aussenschale (1) eine zugehörige und als Führung für den Nocken (7) wirkende Nut (9) angeordnet ist, die eine parallel zur Symmetrieachse der Pfanne verlaufende Zylindermantelfläche aufweist, wobei bei einem Einschieben des Pfannenkörpers (3) in die Aussenschale (1) in Richtung der Pfannenachse der Nocken (7) in die Nut (9) eingreift, bevor der Vorsprung (5) an der Hohlraumwand der Aussenschale (1) zum Aufweiten der Aussenschale zur Anlage kommt.

2.  Endoprothese nach Anspruch 1, dadurch gekennzeichnet, dass die äquatorseitige Begrenzung (6a) der Vertiefung (6) in der Aussenschale (1) senkrecht zur Symmetrieachse der Prothese verläuft, während der zugehörige Abschluss (5r) des Vorsprungs (5) mit dieser Begrenzung (6a) einen spitzen Winkel bildet.

## Claims

1.  An acetabular endoprosthesis comprising an outer shell (1) fixable in the bone and an inner plastics cup (3), the latter having a conical envelope while the outer shell (1) has a cavity (2) adapted to the envelope shape of the cup (3), the latter and the outer shell (1) being retained one in another by a snap fastening (5, 6) such that a projection (5) in the envelope of the cup (3) engages in a recess (6) in the cavity wall, characterised in that a peripherally extending cam (7) which is continuous over the whole periphery is disposed in the cup envelope (8) with an offset towards the pole from the snap fastening (5, 6), the same being disposed near the equator, and an associated groove (9) effective as guide for the cam (7) is disposed in the cavity (2) in the outer shell (1) and has a cylindrical envelope surface which extends parallel to the axis of symmetry of the acetabulum, engagement of the cup (3) in the outer shell (1) in the direction of the cup axis causing the cam (7) to engage in the groove (9) before the projection (5) engages the cavity wall of the outer shell (1) to expand the same.

2. An endoprosthesis according to claim 1, characterised in that that boundary (6a) of the recess (6) which is on the equator side extends in the outer shell (1) perpendicular to the axis of symmetry of the prosthesis while the associated closure (5r) of the projection (5) forms an acute angle with the last-mentioned boundary (6a).

**Revendications**

1. Endoprothèse acétabulaire, constituée d'une coque extérieure (1) ancrée dans l'os et d'un corps de cavité cotyloïde (3) intérieur en matière plastique, le corps de cavité cotyloïde (3) étant pourvu d'une enveloppe conique et la coque extérieure (1) d'une cavité (2) adaptée à la forme de l'enveloppe du corps de cavité cotyloïde (3), et le corps de cavité cotyloïde (3) et la coque extérieure (1) étant maintenus l'un dans l'autre par une fermeture à encliquetage (5, 6) dans laquelle une partie saillante (5) de l'enveloppe du corps de cavité cotyloïde (3) s'emboîte dans un creux (6) de la paroi de la cavité, caractérisée en ce qu'un os (7), s'étendant dans la direction périphérique et continu sur tout le pourtour, est placé dans l'enveloppe (8) du corps de cavité cotyloïde (3), à une certaine distance de la fermeture à encliquetage (5, 6) proche de l'équateur, décalé vers le pôle, et en ce qu'une rainure (9) correspondante et servant de guidage à l'os (7) est ménagée dans la cavité (2) de la coque extérieure (1) et présente une enveloppe cylindrique parallèle à l'axe de symétrie de la cavité cotyloïde, l'os (7) s'emboîtant dans la rainure (9) lorsque le corps de cavité cotyloïde (3) est enfoncé dans la coque extérieure (1), dans la direction de l'axe de la cavité cotyloïde, avant que la partie saillante (5) ne vienne s'appliquer contre la paroi de cavité de la coque extérieure (1) pour élargir celle-ci.

2. Endoprothèse selon la revendication 1, caractérisée en ce que la délimitation (6a), côté équateur, du creux (6) pratiqué dans la coque extérieure (1), est perpendiculaire à l'axe de symétrie de la prothèse, tandis que la fermeture (5r) correspondante de la partie saillante (5) forme un angle aigu avec cette délimitation (6a).

Fig.1

Fig.3

Fig.2